# EUROPEAN PATENT APPLICATION

(11) **EP 3 998 612 A1**
(43) Date of publication of application: **18.05.2022**
(21) Application number: 20836942.1
(22) Date of filing: 28.05.2020
(51) Int. Cl.: G16H 10/20, G16H 80/00

(54) **HEALTH CARE ASSISTANCE DEVICE, METHOD FOR OPERATING SAME, HEALTH CARE ASSISTANCE PROGRAM, AND HEALTH CARE ASSISTANCE SYSTEM**

(30) Priority: 08.07.2019 JP 2019127109
(71) Applicant: FUJIFILM Corporation, Tokyo 106-8620 (JP)
(72) Inventor: SUGAHARA, Masataka, Ashigarakami-gun, Kanagawa 258-8538 (JP)
(74) Representative: Hughes, Andrea Michelle
(86) International application number: PCT/JP2020/021120
(87) International publication number: WO 2021/005909

(57) **Abstract**

Provided is a medical support device, an operation method of a medical support device, a medical support program, and a medical support system capable of performing support for giving an appropriate examination instruction to an examination department.

A medical support device (10) includes a processor (14), in which the processor (14) acquires instruction examination information regarding a type of examination determined by a medical staff, acquires inquiry information generated by voice in an inquiry, acquires correspondence information in which inquiry information and performance examination information regarding the type of examination to be performed are associated with each other in advance, generates estimated examination information in which the type of examination to be performed is estimated based on the inquiry information and the correspondence information, generate a result of comparison of the instruction examination information and the estimated examination information, and controls a notification based on the result of comparison.

## Description

### BACKGROUND OF THE INVENTION

### 1. Field of the Invention

The present invention relates to a medical support device, an operation method of a medical support device, a medical support program, and a medical support system.

### 2. Description of the Related Art

In a medical field, in a scene of a medical care, a medical staff including a doctor obtains various pieces of information for a diagnosis or a treatment by making an inquiry with a patient. For example, the doctor decides a type of examination and a treatment policy based on the information obtained by the inquiry, and gives an examination instruction to an examination department as necessary.

As a method of using the information obtained in the inquiry, a medical support method and a medical support system are disclosed in which a temporary examination is selected based on inquiry data acquired by an inquiry, a secondary examination is selected based on examination data acquired by performing the primary examination (JP2018-5553A). The medical support method and the like disclosed in JP2018-5553A are to reduce a burden on a patient and the like and improve efficiency of a medical care while ensuring accuracy of a diagnosis.

### SUMMARY OF THE INVENTION

Due to the nature of a medical treatment, there are cases in which a doctor or the like decides or confirms a necessary examination for a patient and then wants to give an examination instruction to an examination department. In this case, while the doctor or the like can confirm the examination before giving the examination instruction, it may not be possible to give an appropriate examination instruction due to a human error or the like, resulting in an inappropriate examination instruction.

Regarding the examination instruction, it can be said that the examination instruction is appropriate in a case in which one or more types of examination as instructed by the doctor or the like are suitable for the diagnosis or the treatment of the patient without excess or deficiency. Therefore, examples of the inappropriate examination instruction include a case of an omission of the examination instruction that instructions for all necessary examinations are not given, a case of an excessive examination instruction that an instruction for unnecessary examination is given, and a case of an error of the examination instruction due to a human error in the examination. Examples of the error of the examination instruction include a case in which a wrong examination instruction is given unintentionally by the doctor or the like and a case of an inappropriate examination instruction that the doctor or the like is unaware of another more appropriate examination.

An object of the present invention is to provide a medical support device, an operation method of a medical support device, a medical support program, and a medical support system capable of supporting a medical staff to give an appropriate examination instruction to an examination department.

An aspect of the present invention relates to a medical support device comprising a processor, in which the processor acquires instruction examination information for notifying an examination department of a type of examination determined to be performed on a patient by a medical staff based on an inquiry with the patient, acquires inquiry information generated by analyzing voice uttered during the inquiry with the patient, acquires correspondence information in which the inquiry information and performance examination information regarding the type of examination to be performed on the patient are associated with each other in advance, generates estimated examination information in which the type of examination to be performed on the patient is estimated based on the inquiry information and the correspondence information, compares the instruction examination information with the estimated examination information to generate a result of comparison, and controls a notification based on the result of comparison.

It is preferable that the processor impart an adaptation degree to the type of examination to be performed on the patient, and generate the estimated examination information in which the type of examination of which the adaptation degree is within a range of a specific value is estimated.

It is preferable that in a case in which the instruction examination information and the estimated examination information do not include any common type of examination in the result of comparison, the processor give the notification by a notification content for prompting the medical staff to confirm the instruction examination information.

It is preferable that the processor further give the notification by a notification content including the estimated examination information.

It is preferable that in a case in which the instruction examination information and the estimated examination information include a partially common type of examination in the result of comparison, the processor give the notification by a notification content including the estimated examination information.

It is preferable that in a case in which the instruction examination information and the estimated examination information match in the result of comparison, the processor give a notification regarding the match.

It is preferable that the processor perform a control of acquiring the voice uttered during the inquiry with the patient, and analyze the voice to generate the inquiry information.

It is preferable that the processor control the acquisition of the voice, and control storage of the acquired voice.

It is preferable that the processor start acquisition of the voice in a case of activation.

It is preferable that the processor start acquisition of the voice in a case in which the voice is detected.

It is preferable that the processor store the acquired voice for each patient.

It is preferable that the processor delete the voice after analyzing the voice.

It is preferable that the processor acquire instruction-completed examination information regarding the type of examination determined to be performed on the patient by the medical staff, of which notice to the examination department is completed after the notification is given.

It is preferable that the processor generate a learned model that is learned by associating the inquiry information and the instruction-completed examination information with each other, and perform a control of storing the learned model.

It is preferable that the processor acquire the learned model as the correspondence information.

In addition, another aspect of the present invention relates to an operation method of a medical support device, the method comprising an instruction examination information acquisition step of acquiring instruction examination information for notifying an examination department of a type of examination determined to be performed on a patient by a medical staff based on an inquiry with the patient, an inquiry information acquisition step of acquiring inquiry information generated by analyzing voice uttered during the inquiry with the patient, a correspondence information acquisition step of acquiring correspondence information in which the inquiry information and performance examination information regarding the type of examination to be performed on the patient are associated with each other in advance, an examination estimation step of generating estimated examination information in which the type of examination to be performed on the patient is estimated based on the inquiry information and the correspondence information, a comparison step of comparing the instruction examination information with the estimated examination information to generate a result of comparison, and a notification control step of controlling a notification based on the result of comparison.

In addition, still another aspect of the present invention relates to a medical support program installed in a medical support device, the program causing a computer to realize a function of acquiring instruction examination information for notifying an examination department of a type of examination determined to be performed on a patient by a medical staff based on an inquiry with the patient, a function of acquiring inquiry information generated by analyzing voice uttered during the inquiry with the patient, a function of acquiring correspondence information in which the inquiry information and performance examination information regarding the type of examination to be performed on the patient are associated with each other in advance, a function of generating estimated examination information in which the type of examination to be performed on the patient is estimated based on the inquiry information and the correspondence information, a function of comparing the instruction examination information with the estimated examination information to generate a result of comparison, and a function of controlling a notification based on the result of comparison.

In addition, still another aspect of the present invention relates to a medical support system comprising the medical support device.

According to the present invention, it is possible to support the medical staff to give an appropriate examination instruction to the examination department.

### BRIEF DESCRIPTION OF THE DRAWINGS

Fig. 1 is a block diagram showing a configuration of a medical support device.
Fig. 2 is an explanatory diagram showing a flow of an examination instruction.
Fig. 3 is a block diagram showing a function of the medical support device.
Fig. 4 is a block diagram showing a function of an inquiry information acquisition unit.
Fig. 5 is an explanatory diagram for describing correspondence information.
Fig. 6 is an explanatory diagram showing generation of estimated examination information.
Fig. 7 is an explanatory diagram showing the generation of the estimated examination information using an adaptation degree.
Fig. 8 is an explanatory diagram showing a result of comparison and a notification content in a first case.
Fig. 9 is an explanatory diagram showing a result of comparison and a notification content in a second case.
Fig. 10 is an explanatory diagram showing a result of comparison and a notification content in a third case.
Fig. 11 is an explanatory diagram for describing the notification content.
Fig. 12 is a block diagram showing a function of a medical support device according to another example.
Fig. 13 is a flowchart showing a flow of the examination instruction.

### DESCRIPTION OF THE PREFERRED EMBODIMENTS

A medical support device according to an embodiment of the present invention is used in a scene in which a medical staff gives an examination instruction to an examination department. Therefore, in the present embodiment, the medical support device is installed in a medical examination room or the like in which a doctor makes an inquiry with a patient. In the present specification, the medical staff means a person who can make a decision regarding an examination to be performed on a patient, and examples of the medical staff include a doctor, and a nurse, a clinical technologist, or a radiographer who is a person who can make a decision regarding an examination under an instruction of a doctor or the like. The medical support device is, for example, a computer, such as a personal computer, in which an application program for executing a predetermined function is installed.

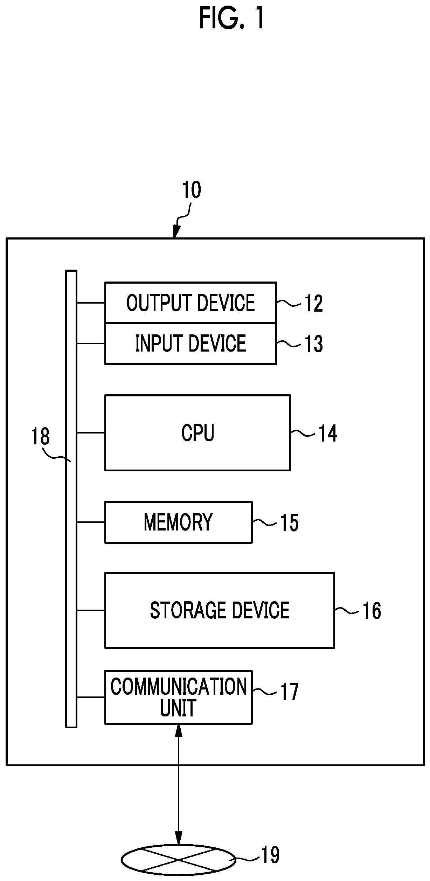

As shown in Fig. 1, a medical support device 10 comprises a central processing unit (CPU) 14 which is a processor, a memory 15, and a storage device 16, and a communication unit 17, in addition to an output device 12, such as a display, and an input device 13, such as a mouse, a microphone, or a keyboard. These units are mutually connected via a data bus 18. The communication unit 17 has a function of communicating with another device or a network 19.

The storage device 16 is a hard disk drive which is built in the medical support device 10 or connected to the medical support device 10 via a cable or the network 19, or a disk array in which a plurality of hard disk drives are mounted. Therefore, the storage device 16 functions as a storage unit. The storage device 16 stores a control program, such as an operating system, various application programs, and various data associated with these programs.

The memory 15 is a work memory for the CPU 14 to execute a process. The CPU 14 comprehensively controls each unit of the medical support device 10 by loading the program stored in the storage device 16 into the memory 15 and executing the process according to the program.

The medical support device 10 may be connected to, via the communication unit 17, an information management server, such as a hospital information system (HIS), a radiology information system (RIS), and the like, which registers and manages information, such as patient information, medical care information, examination information, accounting information, and imaging instruction for image diagnosis, for each patient or an image server, such as a picture archiving and communication system for medical application (PACS), which manages a captured radiation image and information regarding the radiation image. In a case in which the medical support device 10 is connected to these servers, mutual communication of various pieces of information is performed with these servers. In addition, a computer of the medical support device 10 can also be used as a client terminal of these servers.

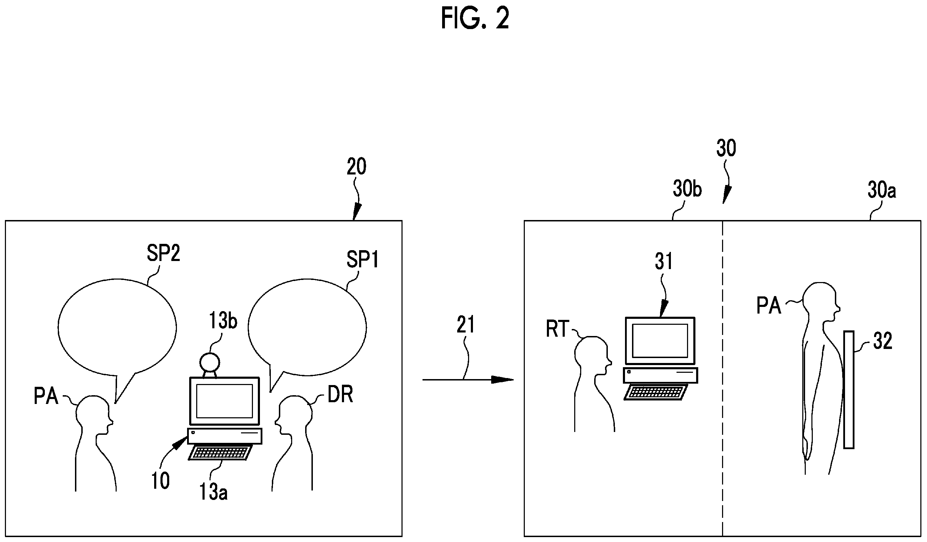

As shown in Fig. 2, the medical support device 10 is installed in the medical examination room 20 and is operated by a person who can make a decision regarding the examination to be performed on the patient, such as a doctor DR. In the medical examination room 20, the doctor DR makes an inquiry by visual examination, auscultation, percussion, palpation, or the like in addition to making a conversation with a patient PA. In the conversation, for example, the doctor DR utters voice SP1 to ask a question, and the patient PA utters voice SP2 to answer the question depending on the voice SP1. The doctor DR decides a diagnosis or a treatment policy during the inquiry, and gives an examination instruction 21 for the patient PA with the support of the medical support device 10, for example, to an image examination room 30 which is an examination department. The image examination room 30 includes an examination room 30a and an operation room 30b. Specifically, the examination instruction 21 is given using the input device 13 of the medical support device 10. The input device 13 includes, for example, a keyboard 13a or a microphone 13b, and these units are collectively referred to as the input device 13.

The examination instruction 21 is given via the RIS (not shown). A client terminal 31 of the RIS is installed in the operation room 30b, and a radiographer RT receives the examination instruction 21 from the client terminal 31. Based on the examination instruction 21, the radiographer RT acquires a medical image of the patient PA in the examination room 30a using, for example, an X-ray imaging device 32.

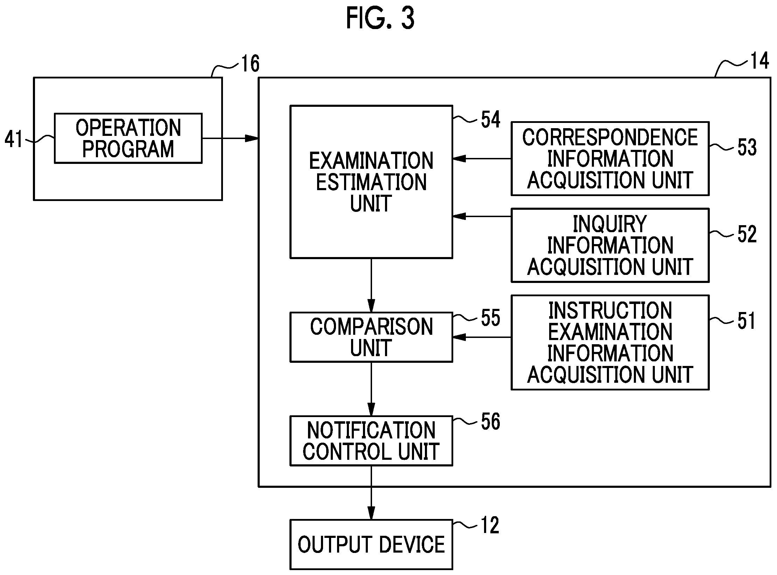

As shown in Fig. 3, in the medical support device 10, the storage device 16 stores an operation program 41. In a case in which the operation program 41 is activated, the CPU 14 cooperates with the memory 15 and the like to function as an instruction examination information acquisition unit 51, an inquiry information acquisition unit 52, a correspondence information acquisition unit 53, an examination estimation unit 54, a comparison unit 55, a notification control unit 56, and the like. Therefore, the medical support device 10 comprises the instruction examination information acquisition unit 51, the inquiry information acquisition unit 52, the correspondence information acquisition unit 53, the examination estimation unit 54, the comparison unit 55, and the notification control unit 56.

The instruction examination information acquisition unit 51 acquires instruction examination information for notifying the image examination room 30 which is the examination department of a type of examination determined to be performed on the patient PA by the doctor DR based on the inquiry with the patient PA. The acquisition of the instruction examination information is performed, for example, by the doctor DR inputting various pieces of information for giving the notification of the type of examination to the input device 13. The inquiry information acquisition unit 52 acquires inquiry information obtained during the inquiry with the patient PA by analyzing the voice uttered during the inquiry with the patient PA. The inquiry information is acquired, for example, by inputting various pieces of information to the input device 13.

The correspondence information acquisition unit 53 acquires correspondence information in which the inquiry information and examination information regarding the type of examination to be performed on the patient PA are associated with each other in advance. The correspondence information is used by, for example, being stored in a storage device. The examination estimation unit 54 generates estimated examination information in which the type of examination to be performed on the patient PA is estimated based on the inquiry information and the correspondence information. The examination estimation unit 54 acquires the inquiry information from the inquiry information acquisition unit 52, and also acquires the correspondence information from the correspondence information acquisition unit 53. The generated estimated examination information is transmitted to the comparison unit 55.

The comparison unit 55 compares the instruction examination information with the estimated examination information and generates a result of comparison. The instruction examination information is acquired from the instruction examination information acquisition unit 51, and the estimated examination information is acquired from the examination estimation unit 54. The generated result of comparison is transmitted to the notification control unit 56. The notification control unit 56 controls the notification based on the result of comparison acquired from the comparison unit 55. In a case in which the notification is given, the notification is given using the output device 12 or the like, such as a display.

Next, each unit and the like provided in the medical support device 10 will be described in detail. The instruction examination information acquired by the instruction examination information acquisition unit 51 is information regarding the type of examination determined to be performed on the patient PA by the doctor DR by the inquiry and the like, and is information for giving the examination instruction by notifying the examination department to perform the type of examination determined to be performed on the patient PA. Therefore, the instruction examination information includes information necessary for the instructed examination department to perform the examination on the patient PA as instructed without any problem. In addition, the instruction examination information may include information indicating that the patient PA does not need the examination as a result of the determination of the doctor DR. Therefore, the instruction examination information may not include any type of examination.

The instruction examination information includes the type of examination performed on the patient PA, an examination condition, and the like. For example, in a case in which the doctor DR determines by the inquiry that an imaging examination and a blood examination with respect to the patient are necessary for the diagnosis or the treatment of the patient PA, the instruction examination information includes information regarding an instruction for acquiring a standing front image of a chest X ray image of which positioning is posterior → anterior (P → A), and an instruction for performing a serological examination in the blood examination. The instruction examination information includes information, such as a name of the type of examination to be performed, but also various pieces of information regarding the examination that may be performed on the patient for the diagnosis, such as the examination condition, in addition to the type of examination.

The doctor DR inputs the instruction examination information to the medical support device 10. In addition, since the medical support device 10 is connected to the HIS and/or the RIS, the instruction examination information input by the doctor DR to the medical support device 10 may be automatically input to the HIS and/or the RIS. An input unit or an input method is not limited, and the instruction examination information is input by, for example, the keyboard 13a or the input device 13, such as the mouse (not shown). In a case in which the mouse is used, for example, a method, such as selecting from an examination list displayed on the display, is used. Note that the instruction examination information is information for the doctor DR to notify or instruct the examination department, but when the instruction examination information is input to the medical support device 10, the instruction is not given to the examination department yet.

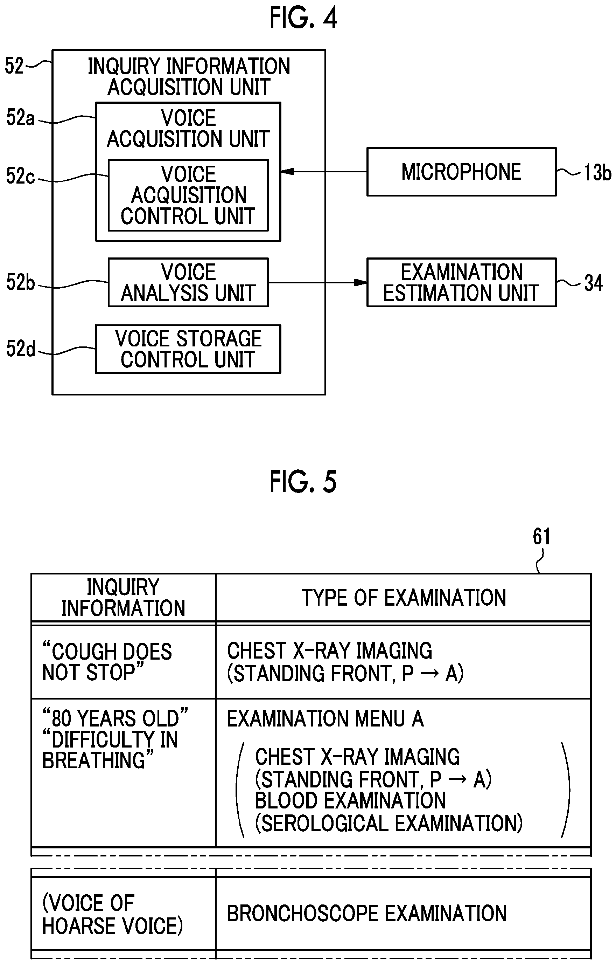

The inquiry information acquisition unit 52 acquires the inquiry information generated by analyzing the voice uttered during the inquiry with the patient PA. As shown in Fig. 4, the inquiry information acquisition unit 52 comprises a voice acquisition unit 52a and a voice analysis unit 52b. The voice acquisition unit 52a performs a control of acquiring the voice uttered during the inquiry. The voice analysis unit 52b analyzes the voice and generates the inquiry information. A data format of the acquired voice is not particularly limited as long as it can be subjected to a process, such as storage or analysis, by the computer.

The voice acquisition unit 52a mainly performs a control of acquiring the voice SP1 uttered by the doctor DR and the voice SP2 uttered by the patient PA during the inquiry. Note that the voice by a person other than the doctor DR and the patient PA, such as an attendant of the patient PA or a nurse, who may speak during the inquiry, may be included.

In the present specification, the inquiry information is information used by the doctor DR to give an appropriate examination instruction to the examination department regarding the type of examination to be performed on the patient PA. Therefore, the inquiry information is information for instructing the examination department to perform an appropriate examination in a case of the diagnosis or the treatment of the patient PA without excess or deficiency, and examples thereof include a subjective symptom of the patient PA and its progress, a medical history of patient PA, and a family health history of the patient PA. In addition, the inquiry information also includes information regarding the patient PA him/herself, such as an age, an occupation, or a family structure of the patient PA. In addition, the inquiry information also includes information based on a statement content of the doctor DR or the nurse regarding the patient PA during the inquiry. For example, the information is information, which is spoken by the doctor DR during the inquiry, regarding a name of a disease with which the patient PA may be infected or a progress so far in a case in which the patient PA is revisited. Therefore, the inquiry information may include information acquired from an electronic medical record of the patient PA.

The inquiry information further includes information other than a content of the conversation during the inquiry, such as a conversation speed, volume, or clarity of words in the statement of the patient PA in the voice SP2 of the patient PA or a time from when the doctor DR asks the question by the voice PA1 until the patient PA responds by the voice SP2. During the inquiry, the doctor DR causes the patient PA to make statement regarding the patient PA including the inquiry information by the statement of the doctor DR. The voice acquisition unit 52a performs a control of acquiring the voice SP1 uttered by the doctor DR and the voice SP2 uttered by the patient PA by these statements.

A voice acquisition unit is not particularly limited. For example, the microphone 13b acquires the voice SP1 spoken by the doctor DR and the voice SP2 spoken by the patient PA, which are uttered during the inquiry. As a procedure for voice acquisition, for example, a voice acquisition button (not shown) for giving an instruction for starting and terminating the voice acquisition is provided in the medical support device 10 or the microphone 13b and the doctor DR operates the voice acquisition button for each inquiry, so that and the voice SP1 and the voice SP2 can be acquired for each patient PA.

The voice analysis unit 52b analyzes the voice including the voice SP1 and the voice SP2 to acquire the inquiry information. As an analysis unit, a known voice recognition unit and/or a natural language processing unit can be used. As the known voice recognition unit and/or the natural language processing unit, a known machine learning technique or a technique other than the machine learning technique can be used. For example, a statement content and information regarding a person who speaks can be acquired by the voice recognition unit using the known machine learning technique as text information, a phrase necessary for giving an appropriate examination instruction for the diagnosis or the treatment of patient PA can be acquired by the natural language processing unit for this text information, and the acquired information can be used as the inquiry information. In addition to the text information, various voice recognition units may acquire the information other than the content of the conversation, such as the conversation speed, the volume, or the clarity of words in the statement of the patient PAbased on the voice SP2 of the patient PA, as the inquiry information. In addition, depending on a type of inquiry information to be acquired, a plurality of the voice recognition units and/or the natural language processing units may be used, and these voice recognition units and/or natural language processing units may be used properly. The obtained inquiry information is transmitted to the examination estimation unit 54.

The correspondence information acquisition unit 53 acquires the correspondence information in which the inquiry information and the performance examination information regarding the type of examination to be performed on the patient PA are associated with each other in advance. The correspondence information is information in which the inquiry information and the performance examination information that is the examination performed on the patient PA who receives the inquiry for which the inquiry information is acquired are associated with each other in advance, but the inquiry information and the performance examination information may be information acquired by the inquiry which is actually performed or assumed. That is, the inquiry information and the performance examination information associated with each other in the correspondence information may each use the assumed information.

The correspondence information need only be information in which the inquiry information and the performance examination information of the examination performed on the patient PA are associated with each other in advance, and a format, a generation method, or the like is not limited. Since the inquiry information is obtained by analyzing the voice, examples thereof may include various formats of voice information, various formats of text information obtained by analyzing the voice, and the like. Therefore, the correspondence information can take various formats depending on the inquiry information. For example, the correspondence information may be created as a table by associating known inquiry information, which is the text information, with the performance examination information of the examination performed on the patient PA. In addition, a learned model created by the examination estimation unit 54 may be used as the correspondence information. This learned model will be described below. Note that the inquiry information used in the correspondence information may include a plurality of pieces of information constituting the inquiry information, and the performance examination information may also be an examination menu including a plurality of types of examination.

As the correspondence information, a plurality of pieces of correspondence information may be used properly such that preferable estimated examination information can be obtained depending on various conditions. Therefore, the learned models which are learned differently may be used properly depending on various conditions. For example, the preferable estimated examination information may be obtained by properly using the learned models which are the correspondence information depending on the age of the patient PA. Note that the preferable estimated examination information means that the type of examination included in the estimated examination information and the appropriate examination instruction for the patient PA match. Therefore, it can be said that the inquiry information and/or the correspondence information for generating the estimated examination information are preferable in a case in which the type of examination included in the estimated examination information is suitable for the diagnosis or the treatment of the patient without excess or deficiency.

As shown in Fig. 5, correspondence information 61 is, for example, the information in which the inquiry information and the performance examination information of the examination performed on the patient PA who receives the inquiry from which the inquiry information is acquired are associated with each other. The correspondence information 61 may be correspondence information in which one or a plurality of pieces of inquiry information one or a plurality of types of examination or one or a plurality of examination menus are associated with each other. In the correspondence information 61, for example, in a case in which the inquiry information is a phrase "cough does not stop" based on the statement of the patient PA during the inquiry, "chest X-ray imaging (standing front, P → A)" is associated as the type of examination based on the performance examination information. In addition, in a case in which the inquiry information is phrases "80 years old" and "difficulty in breathing" based on the statement to the patient PA during the inquiry, "examination menu A" is associated as the type of examination based on the performance examination information, and a content of the "examination menu A" is "chest X-ray imaging (standing front, P → A)" and "blood examination (serological examination)". In addition, in a case in which the inquiry information is not a phrase included in the statement of the patient PA, and is a voice state of the patient PA, for example, the voice of a hoarse voice, "bronchoscope examination" is associated as the type of examination based on the performance examination information.

A unit by which the correspondence information acquisition unit 53 acquires the correspondence information is not limited. For example, the correspondence information is stored in the storage device (not shown) in advance, and the correspondence information acquisition unit 53 can acquire the correspondence information stored in the storage device.

The examination estimation unit 54 generates the estimated examination information in which the type of examination to be performed on the patient PA is estimated based on the inquiry information and the correspondence information. As described above, the examination estimation unit 54 is connected to the inquiry information acquisition unit 52, and the examination estimation unit 54 receives the inquiry information acquired by the inquiry information acquisition unit 52. In addition, the examination estimation unit 54 is connected to the correspondence information acquisition unit 53, and the examination estimation unit 54 receives the correspondence information acquired by the correspondence information acquisition unit 53. The examination estimation unit 54 estimates an appropriate type of examination based on the inquiry information received from the inquiry information acquisition unit 52 from the inquiry information received from the inquiry information acquisition unit 52 and the correspondence information received from the correspondence information acquisition unit 53, and generates the estimated examination information including the appropriate type of examination. The appropriate type of examination is not limited to one type of examination, and may include a plurality of types of examination. Note that since the estimated examination information is information in which the type of examination to be performed on the patient is estimated, the estimated examination information is information including the type of examination, but also includes information in which the examination is estimated not to be necessary. In addition, each information included in the inquiry information and/or the correspondence information used by the examination estimation unit 54 may be weighted.

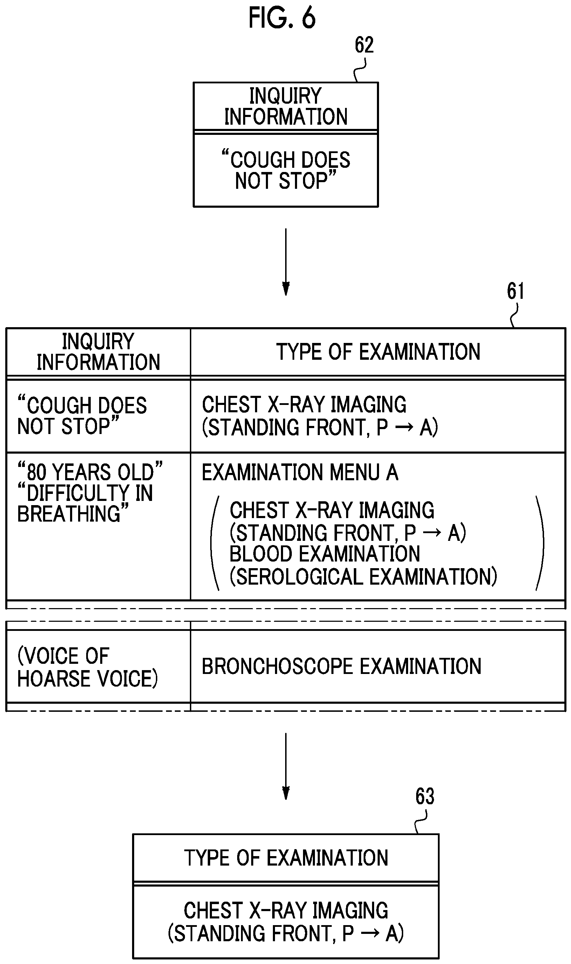

As shown in Fig. 6, for example, in a case in which the inquiry information 62 received from the inquiry information acquisition unit 52 includes the phrase "cough does not stop" acquired by analyzing the voice SP2 of the patient PA, the examination estimation unit 54 estimates estimated examination information 63 that the type of examination is "chest X-ray imaging (standing front, P → A)" based on the correspondence information 61.

In addition, the examination estimation unit 54 may impart an adaptation degree to the type of examination to be performed on the patient PA, and may generate the estimated examination information in which the type of examination to which the adaptation degree equal to or larger than a specific value is imparted is estimated. The adaptation degree imparted by the examination estimation unit 54 is a numerical value corresponding to a probability that the estimated type of examination is performed on the patient PA in a case in which the examination estimation unit 54 estimates the type of examination performed on the patient PA based on the inquiry information and the correspondence information. A method of imparting the adaptation degree is not limited, and for example, the type of examination to which the adaptation degree is imparted may be estimated by a machine learning technique or the like using the learned model as the correspondence information. This learned model will be described below. Since the adaptation degree is imparted to the type of examination, the type of examination of which the adaptation degree is within a range of the specific value may be selected to generate the estimated examination information. The specific value of adaptation degree can be set.

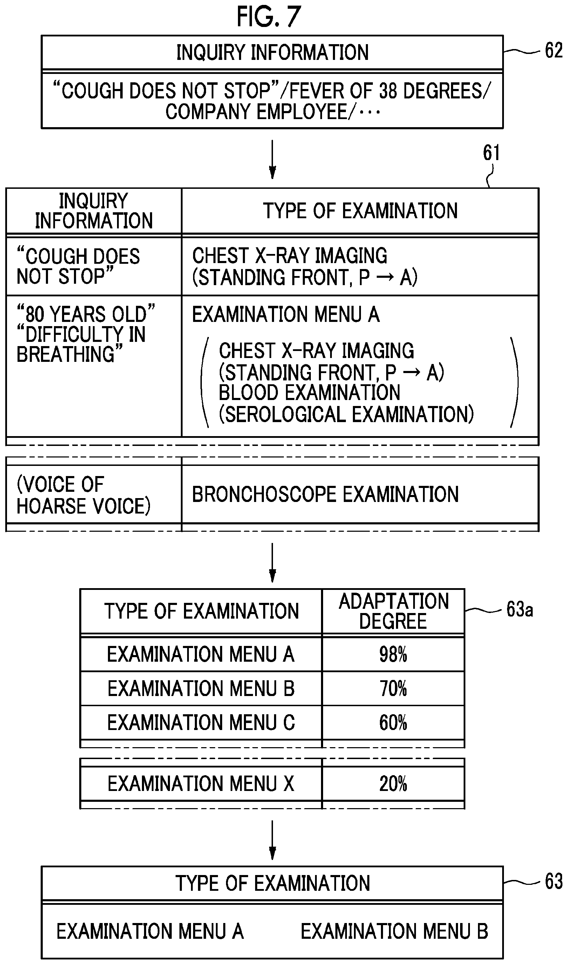

As shown in Fig. 7, for example, in a case in which the inquiry information 62 received from the inquiry information acquisition unit 52 includes the phrase "cough does not stop" acquired by analyzing the voice SP2 of the patient PA, information of a body temperature, such as "fever of 38 degrees", and personal information of the patient PA, such as information that the patient PA is "company employee", the examination estimation unit 54 generates an estimated examination list 63a in which the types of examination to which the adaptation degree is imparted are estimated based on the correspondence information 61. In a case in which the estimated examination information 63 is generated under a condition that the adaptation degree of the type of examination is equal to or larger than 70%, the estimated examination information 63 is generated from the "examination menu A" in which the adaptation degree is "98%" and an "examination menu B" in which the adaptation degree is "70%" in the estimated examination list 63a.

The comparison unit 55 compares the instruction examination information with the estimated examination information and generates the result of comparison. As described above, since the instruction examination information is the information for notifying the examination department of the type of examination determined to be performed on the patient PA by the doctor DR, the instruction examination information includes the information regarding the type of examination. In addition, since the estimated examination information is the information in which the type of examination to be performed on the patient PA is estimated, the estimated examination information includes the information regarding the type of examination. Therefore, the result of comparison is a result of comparison regarding the type of examination.

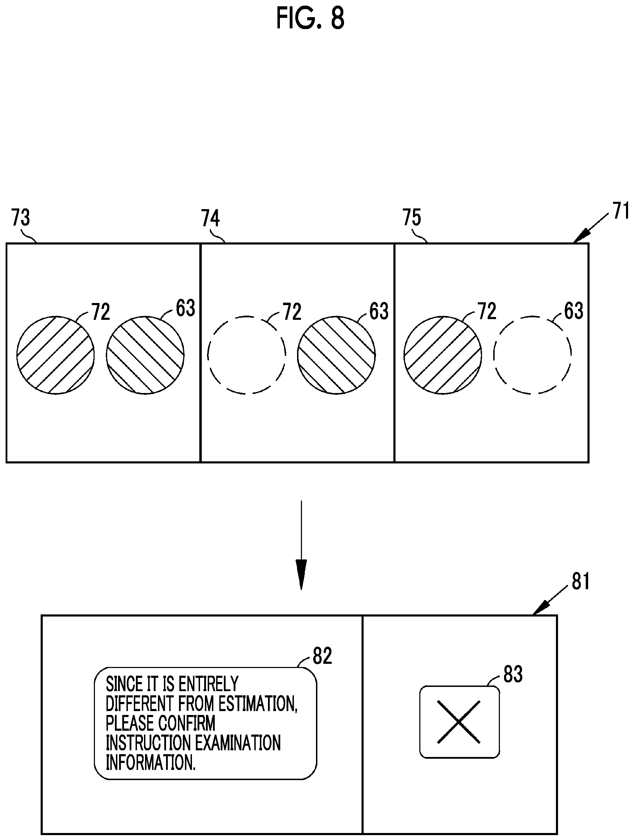

The result of comparison includes cases described below. First, as shown in Fig. 8, there is a case in which instruction examination information 72 and the estimated examination information 63 do not include any common type of examination in a result of comparison 71. That is, the type of examination determined to be performed on the patient PA by the doctor DR is not included in the type of examination estimated by the examination estimation unit 54. In this case, there are a case in which the type of examination is included in the instruction examination information 72 and the type of examination is included in the estimated examination information 63 (case A73), a case in which the examination is determined not to be necessary in the instruction examination information 72, whereas the estimated examination estimation unit 54 determines that the examination is necessary, so that the type of examination is included in the estimated examination information 63 (case B74), and a case in which the examination estimation unit 54 estimates that the examination is not necessary and the information regarding examination is not included in the estimated examination information 63, whereas the examination is determined to be necessary in the instruction examination information 72 (case C75). Note that, in Figs. 8 to 10, a solid circle indicates a set with the type of examination as an element, and hatching with diagonal lines indicates that there is the type of examination which is an element of the set. A dashed circle indicates that there is no type of examination and set.

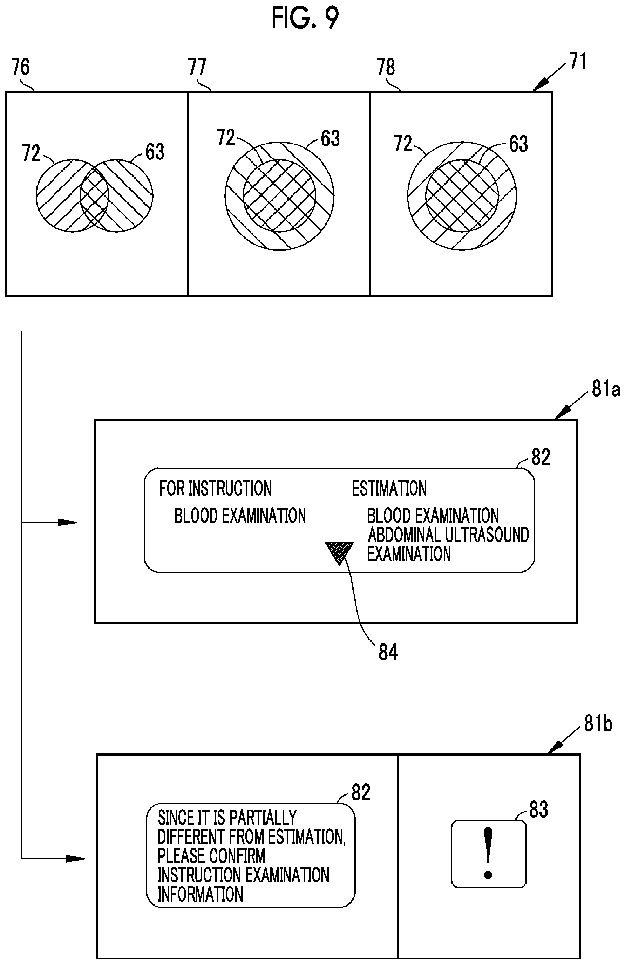

Secondly, as shown in Fig. 9, there is a case in which the estimated examination information 63 and the instruction examination information 72 include a partially common type of examination in the result of comparison 71. In this case, there are a case in which a part of the types of examination determined to be performed on the patient PA by the doctor DR is included in the types of examination estimated by the examination estimation unit 54, but the others are not common (case D76), a case in which all of the types of examination determined to be performed on the patient PA by the doctor DR are included in the types of examination estimated by the examination estimation unit 54, but the other types of examination are also included in the estimated types of examination (case E77), and all of the types of examination estimated by the examination estimation unit 54 are in the types of examination determined to be performed on the patient PA by the doctor DR, but the other types of examination are included in the types of examination determined to be performed by the doctor DR (case F78).

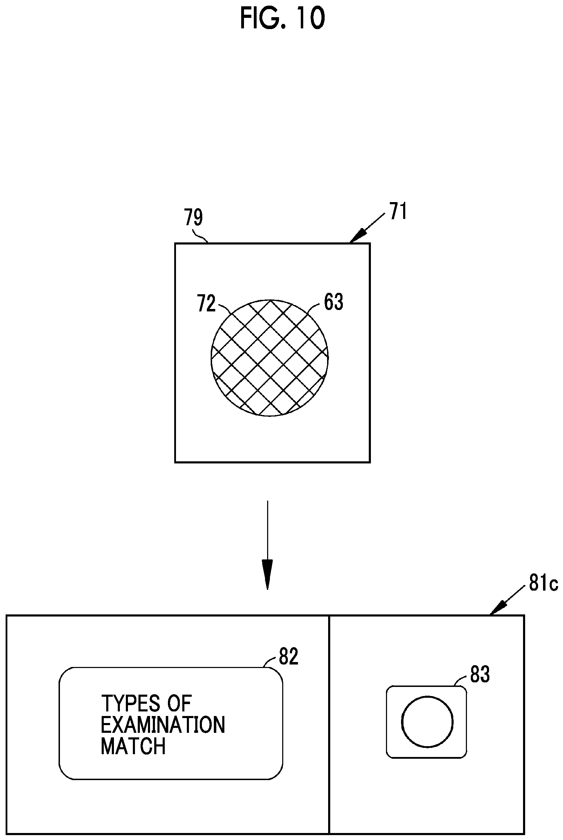

Thirdly, as shown in Fig. 10, there is a case in which the estimated examination information 63 and the instruction examination information 72 match in the result of comparison 71 (case G79). That is, it is a case in which the type of examination determined to be performed on the patient PA by the doctor DR and the type of examination estimated by the examination estimation unit 54 match without excess or deficiency.

The notification control unit 56 controls the notification based on the result of comparison 71. The control of the notification means to decide the matters for executing the notification, such as a control of the presence or absence of a notification with or without a notification, a control of notification contents in a case of the notification, a control of the number of notifications in a case of the notification, or a control of the other party in a case of the notification. These matters can be changed by setting in some cases.

In the present embodiment, the notification is given to the doctor DR or the like who creates the instruction examination information 72 by using the medical support device 10. A notification unit is not limited as long as it can recognize that the notification is given to the doctor DR. For example, in the case of the notification by the output device 12 of the medical support device 10, the notification is given to the doctor DR by displaying text or image on a display 12a which is the output device 12.

The notification control unit 56 decides the notification contents based on the result of comparison 71 and controls the notification. For example, as shown in Fig. 8, in a case in which the instruction examination information 72 and the estimated examination information 63 do not include any common type of examination in the result of comparison 71 (case A73, case B74, or case C75), the notification control unit gives the notification to the doctor DR by notification contents 81 for prompting the confirmation of the instruction examination information 72. The notification contents 81 for prompting the confirmation of the instruction examination information 72 need only be the notification contents as long as the doctor DR is prompted to confirm the instruction examination information 72 and the like including the type of examination determined by him/herself, and examples thereof include a text display 82 for prompting the confirmation of the instruction examination information 72, such as "since it is entirely different from estimation, please confirm instruction examination information", or an image display 83 with a symbol "×" indicating a warning. These text display 82 or the image display 83 is displayed on a part or all of the display.

In addition, for example, as shown in Fig. 9, in a case in which the estimated examination information 63 and the instruction examination information 72 include a partially common type of examination in the result of comparison 71 (case D76, case E77, or case F78), the notification control unit 56 may give, to the doctor DR, the notification by notification contents 81a including the estimated examination information 63 or may give the notification by notification contents 81b for prompting the confirmation of the instruction examination information 72. The notification contents 81a including the estimated examination information 63 as the text display 82 need only be the notification contents by which the doctor DR can recognize a difference between the instruction examination information 72 including the type of examination determined by him/herself and the estimated examination information 63 by recognizing the type of examination of the estimated examination information 63. For example, in the text display 82 in the notification contents 81a, "blood examination" is displayed in the column displayed as "for instruction", and it can be recognized that the instruction examination information 72 includes "blood examination" as the type of examination. On the other hand, "blood examination" and "abdominal ultrasound examination" are described in the column displayed as "estimation", and it can be recognized that the estimated examination information 63 includes "blood examination" and "abdominal ultrasound examination" as the type of examination. Note that, for example, in a case in which the number of types of examination is three or more, the hidden text display 82 can be shown by scrolling by giving an instruction for an inverted triangular arrow 84 displayed on the text display 82 in the notification contents 81a with the mouse or the like.

In addition, for example, the notification contents 81b can be a text display 82b or the like for prompting the confirmation of the instruction examination information 72. Note that examples of the notification contents 81 for prompting the confirmation of the instruction examination information 72 include the text display 82 for prompting the confirmation of the instruction examination information 72, such as "since it is partially different from estimation, please confirm instruction examination information", or the image display 83 with a symbol "!" indicating a warning. The notification contents 81 can be changed by setting.

In addition, for example, as shown in Fig. 10, in a case in which the instruction examination information 72 and the estimated examination information 63 match in the result of comparison 71 (case G79), the notification regarding the match is given. The notification contents 81 of the notification regarding the match need only be the notification contents by which the doctor DR can recognize that the instruction examination information 72 including the type of examination determined by him/herself and the estimated examination information 63 match, and examples thereof include the text display 82, such as "types of examination match", or the image display 83 with a symbol "o" indicating the match. By giving the notification with the fact that the estimated examination information 63 and the instruction examination information 72 match as the notification contents 81, the doctor DR can recognize the type of examination determined to be performed on the patient PA by him/herself and the type of examination estimated by the examination estimation unit 54 match without excess or deficiency.

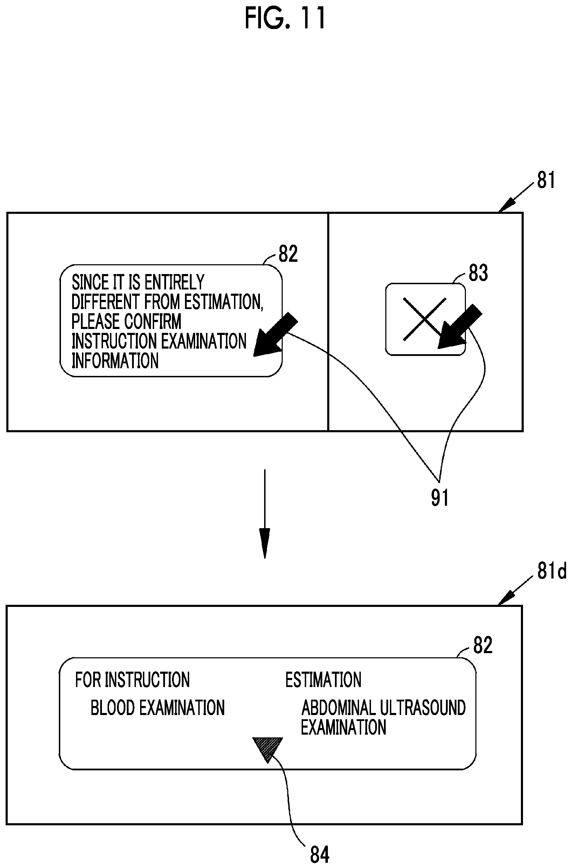

Note that, as shown in Fig. 11, in a case in which the instruction examination information 72 and the estimated examination information 63 do not include any common type of examination in the result of comparison 71, the notification may be given by the notification contents including the estimated examination information 63. For example, in a case in which the text display 82 for prompting the confirmation of the instruction examination information 72, such as "since it is entirely different from estimation, please confirm instruction examination information", or the image display 83 with the symbol "×" indicating a warning is displayed on the display, by clicking the text display 82 or the image display 83 with a cursor 91, notification contents 81d by the text display 82 in which the information regarding the type of examination of the instruction examination information 72 and the information regarding the type of examination of the estimated examination information 63 are described in parallel can be displayed on the display.

As described above, in the medical support device 10 according to the embodiment of the present invention, in a case in which the doctor DR gives the examination instruction to the examination department, the notification is given based on the result of comparison between the estimated examination information 63 estimated by using the voice during the inquiry and the instruction examination information 72, so that it is possible to check the type of examination determined by him/herself and receive suggestions without labor. Therefore, in a case in which the doctor DR gives the examination instruction by him/herself, it is possible to suppress an error in the examination instruction, and it is possible to prevent re-performing of the examination from or performing of a wrong examination. In addition, since the estimated examination information 63 proposes candidates for the examination instructions that the doctor DR does not recognize, the doctor DR can recognize a better type of examination for the patient PA, or a better imaging method in a case of medical imaging examination. Therefore, the medical support device 10 according to the embodiment of the present invention can be suitably used for the education of the doctor DR. The doctor DR gives the examination instruction to the examination department after receiving the notification regarding the instruction examination information 72. Therefore, the doctor DR can give the appropriate examination instruction to the examination department with the support of the medical support device 10.

Note that the inquiry information acquisition unit 52 that acquires the inquiry information may comprise a voice acquisition control unit 52c and a voice storage control unit 52d (see Fig. 4). The voice acquisition control unit 52c controls the acquisition of the voice. In addition, the voice storage control unit 52d controls storage of the acquired voice. Note that, specifically, as the voice acquisition control unit 52c and the voice storage control unit 52d, known units can used.

The voice acquisition control unit 52c realizes the control of acquiring the voice uttered during the inquiry by performing various controls, such as an acquisition unit of the voice. The voice may be acquired by the doctor DR or the like manually giving the instruction for starting and terminating the acquisition of the voice, but it is preferable to automatically acquire the voice. In this case, the control of acquiring the voice is performed, for example, such that the acquisition of the voice is automatically started and the acquisition of the voice is automatically terminated. As a method of automatically starting the acquisition of the voice, for example, the voice acquisition control unit 52c can start the acquisition of the voice in a case in which the voice is detected. In addition, as a method of automatically terminating the acquisition of the voice, for example, the voice acquisition control unit 52c can terminate the acquisition of the voice in a case in which a state without the voice continues for a specific period.

In a case in which the voice acquisition control unit 52c automatically starts the acquisition of the voice, the voice acquisition control unit 52c may start the acquisition of the voice by a specific trigger or switch for starting the acquisition of the voice. For example, in a case in which the activation of the medical support device 10 is used as a trigger for the acquisition of the voice, the acquisition of the voice is automatically started in a case in which the medical support device 10 is activated. In addition, in a case in which the detection of the voice which is the human voice during the inquiry is used as a trigger, the acquisition of the voice is automatically started in a case in which the voice which is the human voice is detected during the inquiry. In addition, in a case in which the detection of the voice of a specific person whose voice is registered during the inquiry is used as a trigger, for example, the acquisition of the voice is automatically started in a case in which the voice of the doctor DR is detected. On the other hand, in a case in which the detection of the voice of a person other than the specific person or a person whose voice is not registered is used as a trigger, the voice of the patient PA can be recognized during the inquiry, so that the acquisition of the voice can be automatically started for each patient. In addition, in a case in which a specific phrase is extracted by analyzing the voice as a trigger, the voice analysis unit 52b analyzes the voice that utters the specific phrase, such as "recording start" regardless of the specific person, and the acquisition of the voice is automatically started in a case in which the phrase "recording start" is extracted.

By these controls by the voice acquisition control unit 52c, for example, the voice uttered during the inquiry can be acquired without the doctor DR or the like manually giving the instruction for the voice acquisition. Note that the specific phrase can be set by the voice acquisition control unit 52c. Note that the voice acquisition control unit 52c starts and/or terminates the acquisition of the voice, as well as controls when the acquired voice is transmitted to the voice analysis unit 52b, for example. In this way, the voice acquisition control unit 52c can prevent forgetting to acquire the voice. In addition, the voice acquisition control unit 52c controls the cooperation with the voice analysis unit 52b, so that various voice acquisition controls can be performed.

The voice storage control unit 52d controls the storage of the acquired voice. For example, the voice storage control unit 52d may store the acquired voice for each patient PA by the cooperation with the voice analysis unit 52b. In this case, in a case in which the acquired voice is analyzed and the phrase of the personal information of the patient PA, such as the name of the patient PA or a medical examination card number, is extracted, the voice can be stored in a file for each patient PA. Note that the storage of the voice may be managed by the RIS or the like in conjunction with the electronic medical record or the like.

Note that the voice acquisition control unit 52c may cooperate with the voice analysis unit 52b to perform a control of deleting the voice after analyzing the voice, for example. As a result, a storage unit that stores the voice can be saved.

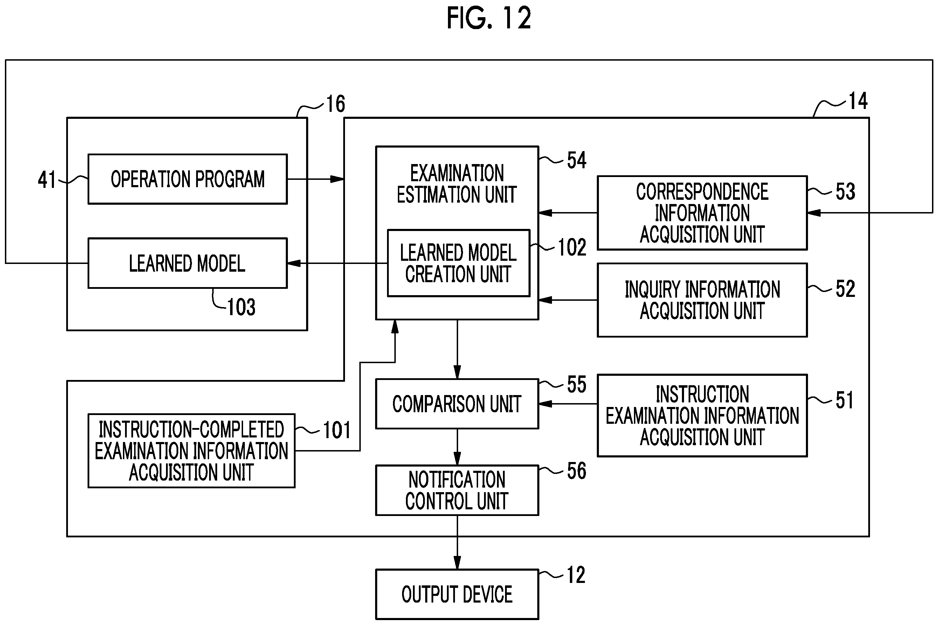

Note that, as shown in Fig. 12, an instruction-completed examination information acquisition unit 101 that acquires instruction-completed examination information regarding the type of examination determined to be performed on the patient PA by the doctor DR, of which notice to the examination department is completed after the notification is given may be provided. The instruction-completed examination information includes information regarding the type of examination determined to be performed on the patient PA by the doctor DR. The instruction-completed examination information includes the type of examination re-determined by the doctor DR after the notification by the medical support device 10 is given, and thus it is the appropriate examination instruction in which the omission of the examination instruction, the excessive examination instruction, the error of the examination instruction, or an inappropriate examination instruction is suppressed.

The instruction-completed examination information is transmitted to the examination estimation unit 54. The examination estimation unit 54 may comprise a learned model creation unit 102. The learned model creation unit 102 is connected to the inquiry information acquisition unit 52 and the instruction-completed examination information acquisition unit 101, and acquires the inquiry information and the instruction-completed examination information, respectively. Then, a learned model 103 learned by associating the inquiry information and the instruction-completed examination information with each other is generated. The generated learned model 103 is stored in a storage device 16 that is the storage unit.

The learned model 103 stored in the storage device 16 is acquired by the correspondence information acquisition unit 53 as the correspondence information. Therefore, the instruction-completed examination information is the performance examination information in the correspondence information. As a result, it is possible to generate the estimated examination information in which the type of examination is estimated with higher accuracy. In addition, regarding the adaptation degree, the adaptation degree can be imparted to the type of examination with higher accuracy. In addition, for example, even in a case in which criteria of the examination to be performed are different for each medical facility, it is possible to estimate the type of examination suitable for each medical facility.

In addition, a medical support system comprising the medical support device 10 comprises the medical support device 10 described above. Moreover, the medical support system may comprise another device connected to the medical support device 10.

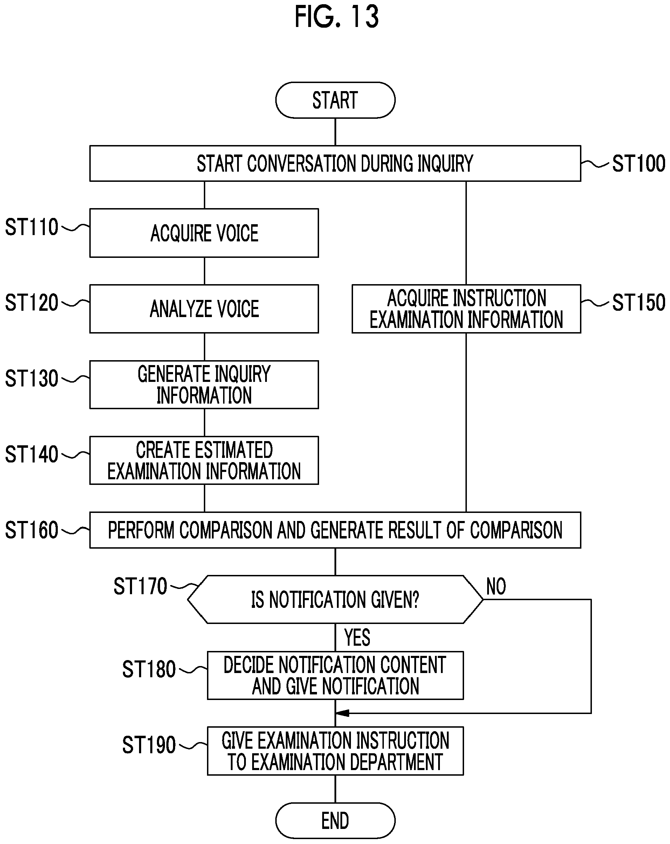

Next, an operation of the configuration described above will be described with reference to the flowchart of Fig. 13. First, during the inquiry, the conversation between the doctor DR and the patient PA is started (step ST100). The voice acquisition unit 52a performs a control of automatically acquiring the voice SP1 uttered by the doctor DR and the voice SP2 uttered by the patient PA (step ST110). The acquired voice SP1 and the acquired voice SP2 are analyzed by the voice analysis unit 52b (step ST120). The inquiry information is generated by the analysis (step ST130). The generated inquiry information is acquired by the inquiry information acquisition unit 52 and transmitted to the examination estimation unit 54. The examination estimation unit 54 generates the estimated examination information (step ST140).

In parallel with a flow of the control of acquiring the voice SP1 and the voice SP2 by the voice acquisition unit 52a during the inquiry, the doctor DR inputs the instruction examination information regarding the type of examination determined to be performed on the patient to the medical support device 10, and the instruction examination information acquisition unit 51 acquires the instruction examination information (step ST150). The comparison unit 55 compares the inquiry information with the estimated examination information and generates the result of comparison (step ST160). Based on the result of comparison, the notification control unit 56 controls whether or not to give the notification (step ST170). In a case in which the notification is given (YES in step ST170), the notification contents are decided and the notification is given (step ST180). In a case in which the notification is not given, the examination instruction is given to the examination department by the instruction examination information without the notification, and in a case in which the notification is given, the examination instruction is given to the examination department by the instruction examination information, which is appropriately corrected by the notification or the confirmation of the notification contents (step SP190). Therefore, the doctor DR can give the appropriate examination instruction to the examination department.

In the embodiment described above, hardware structures of processing units which execute various processes, such as the instruction examination information acquisition unit 51, the inquiry information acquisition unit 52, the correspondence information acquisition unit 53, the examination estimation unit 54, the comparison unit 55, the notification control unit 56, the instruction-completed examination information acquisition unit 101, and the learned model creation unit 102, are various processors as follows. The various processors include a central processing unit (CPU), which is a general-purpose processor that executes software (program) and functions as various processing units, a programmable logic device (PLD) that is a processor of which a circuit configuration can be changed after manufacture, such as a field programmable gate array (FPGA), and a dedicated electric circuit (such as an application specific integrated circuit (ASIC) and a graphical processing unit (GPU)), which is a processor having a circuit configuration that is designed for exclusive use in order to execute various processes.

One processing unit may be configured by one of these various processors, or a combination of two or more processors of the same type or different types (for example, a plurality of FPGAs, a combination of a CPU and a FPGA, or a combination of a GPU and a CPU). In addition, a plurality of the processing units may be configured by one processor. As an example of configuring the plurality of processing units with one processor, first, as represented by a computer, such as a client or a server, there is an aspect in which one processor is configured by a combination of one or more CPUs and the software and the processor functions as the plurality of processing units. Second, as represented by a system on chip (SoC), there is an aspect in which a processor is used in which the functions of the entire system including the plurality of processing units are realized by a single integrated circuit (IC) chip. In this way, the various processing units are configured by one or more of various processors described above as the hardware structures.

Moreover, the hardware structures of these various processors is, more specifically, an electric circuit (circuitry) having an aspect in which the circuit elements such as semiconductor elements are combined.

It is needless to say that the present invention is not limited to the embodiment described above, various configurations can be adopted as long as the configuration does not deviate from the gist of the present invention. Moreover, the present invention is applied to a storage medium that stores the program, in addition to the program.

### Explanation of References

10: medical support device
12: output device
13: input device
13a: keyboard
13b: microphone
14: CPU
15: memory
16: storage device
17: communication unit
18: data bus
19: network
20: medical examination room
21: examination instruction
30: image examination room
30a: examination room
30b: operation room
31: client terminal
32: X-ray imaging device
41: operation program
51: instruction examination information acquisition unit
52: inquiry information acquisition unit
52a: voice acquisition unit
52b: voice analysis unit
52c: voice acquisition control unit
52d: voice analysis unit
53: correspondence information acquisition unit
54: examination estimation unit
55: comparison unit
56: notification control unit
61: correspondence information
62: inquiry information
63: estimated examination information
63a: estimated examination list
71: result of comparison
72: instruction examination information
73: case A
74: case B
75: case C
76: case D
77: case E
78: case F
79: case G
81, 81a, 81b, 81c, 81d: notification content
82: text display
83: image display
84: arrow
91: cursor
101: instruction-completed examination information acquisition unit
102: learned model creation unit
103: learned model
PA: patient
DR: doctor
RT: radiographer
SP1: voice
SP2: voice
ST100 to ST190: step

## Claims

1. A medical support device comprising:
a processor,
wherein the processor
acquires instruction examination information for notifying an examination department of a type of examination determined to be performed on a patient by a medical staff based on an inquiry with the patient,
acquires inquiry information generated by analyzing voice uttered during the inquiry with the patient,
acquires correspondence information in which the inquiry information and performance examination information regarding the type of examination to be performed on the patient are associated with each other in advance,
generates estimated examination information in which the type of examination to be performed on the patient is estimated based on the inquiry information and the correspondence information,
compares the instruction examination information with the estimated examination information to generate a result of comparison, and
controls a notification based on the result of comparison.

2. The medical support device according to claim 1,
wherein the processor
imparts an adaptation degree to the type of examination to be performed on the patient, and
generates the estimated examination information in which the type of examination of which the adaptation degree is within a range of a specific value is estimated.

3. The medical support device according to claim 1 or 2,
wherein in a case in which the instruction examination information and the estimated examination information do not include any common type of examination in the result of comparison, the processor gives the notification by a notification content for prompting the medical staff to confirm the instruction examination information.

4. The medical support device according to claim 3,
wherein the processor further gives the notification by a notification content including the estimated examination information.

5. The medical support device according to claim 1 or 2,
wherein in a case in which the instruction examination information and the estimated examination information include a partially common type of examination in the result of comparison, the processor gives the notification by a notification content including the estimated examination information.

6. The medical support device according to claim 1 or 2,
wherein in a case in which the instruction examination information and the estimated examination information match in the result of comparison, the processor gives a notification regarding the match.

7. The medical support device according to any one of claims 1 to 6,
wherein the processor
performs a control of acquiring the voice uttered during the inquiry with the patient, and
analyzes the voice to generate the inquiry information.

8. The medical support device according to claim 7,
wherein the processor
controls the acquisition of the voice, and
controls storage of the acquired voice.

9. The medical support device according to claim 8,
wherein the processor starts acquisition of the voice in a case of activation.

10. The medical support device according to claim 8,
wherein the processor starts acquisition of the voice in a case in which the voice is detected.

11. The medical support device according to any one of claims 8 to 10,
wherein the processor stores the acquired voice for each patient.

12. The medical support device according to any one of claims 8 to 11,
wherein the processor deletes the voice after analyzing the voice.

13. The medical support device according to any one of claims 1 to 12,
wherein the processor acquires instruction-completed examination information regarding the type of examination determined to be performed on the patient by the medical staff, of which notice to the examination department is completed after the notification is given.

14. The medical support device according to claim 13,
wherein the processor
generates a learned model that is learned by associating the inquiry information and the instruction-completed examination information with each other, and
performs a control of storing the learned model.

15. The medical support device according to claim 14,
wherein the processor acquires the learned model as the correspondence information.

16. An operation method of a medical support device, the method comprising:
an instruction examination information acquisition step of acquiring instruction examination information for notifying an examination department of a type of examination determined to be performed on a patient by a medical staff based on an inquiry with the patient;
an inquiry information acquisition step of acquiring inquiry information generated by analyzing voice uttered during the inquiry with the patient;
a correspondence information acquisition step of acquiring correspondence information in which the inquiry information and performance examination information regarding the type of examination to be performed on the patient are associated with each other in advance;
an examination estimation step of generating estimated examination information in which the type of examination to be performed on the patient is estimated based on the inquiry information and the correspondence information;
a comparison step of comparing the instruction examination information with the estimated examination information to generate a result of comparison; and
a notification control step of controlling a notification based on the result of comparison.

17. A medical support program installed in a medical support device, the program causing a computer to realize:
a function of acquiring instruction examination information for notifying an examination department of a type of examination determined to be performed on a patient by a medical staff based on an inquiry with the patient;
a function of acquiring inquiry information generated by analyzing voice uttered during the inquiry with the patient;
a function of acquiring correspondence information in which the inquiry information and performance examination information regarding the type of examination to be performed on the patient are associated with each other in advance;
a function of generating estimated examination information in which the type of examination to be performed on the patient is estimated based on the inquiry information and the correspondence information;
a function of comparing the instruction examination information with the estimated examination information to generate a result of comparison; and
a function of controlling a notification based on the result of comparison.

18. A medical support system comprising:
the medical support device according to any one of claims 1 to 15.
